# EUROPÄISCHE PATENTSCHRIFT

(11) **EP 2 848 240 B1**
(45) Veröffentlichungstag und Bekanntmachung des Hinweises auf die Patenterteilung: **30.11.2016**
(21) Anmeldenummer: 14181108.3
(22) Anmeldetag: 15.08.2014
(51) Int. Cl.: A61K 8/37, A61K 8/49, A61Q 17/04, A61K 8/85

(54) **Leistungsfähiges Sonnenschutzmittel zur Anwendung auf nasser Haut**
Powerful sunscreens for use on wet skin
Écrans solaires puissants pour l'utilisation sur la peau mouillée

(30) Priorität: 12.09.2013 DE 102013218295
(43) Veröffentlichungstag der Anmeldung: 18.03.2015
(73) Patentinhaber: Beiersdorf AG, 20253 Hamburg (DE)
(72) Erfinder: Johns, Nicole, 22958 Kuddewörde (DE); Blohm, Alexandra, 22607 Hamburg (DE)

(56) Entgegenhaltungen:
- EP-A1- 2 636 401
- EP-A2- 1 180 359
- DE-U1-202013 001 754
- Anonymous: "CosmoSurfTM CE Series High Definiton Polymers - Technical Data Sheet", CosmoSurf , 1. April 2010 (2010-04-01), Seiten 1-8, XP055113562, Gefunden im Internet: URL:http://www.cossma.com/fileadmin/all/co ssma/Archiv/ProductInfo/COS1103_44_Brennta gSiltecCosmosurf_CE_Brochure_2010.pdf [gefunden am 2014-04-10]
- Anonymous: "REDUCING COSTS AND IMPROVINGSENSORIAL PROPERTIES OFSUNSCREEN PRODUCTS", SIGMA , 2010, XP002734440, Gefunden im Internet: URL:http://chemagent.ru/component/flexicon tent/download/1395/1474/19. [gefunden am 2014-01-14]
- Anonymous: "Alkyl Benzoate Finsolv TPP", Innospec , 1. April 2010 (2010-04-01), XP055133019, Gefunden im Internet: URL:http://tempo.ca/wp-content/uploads/201 3/06/FINSOLV-TPP-TDS-10-04-01.pdf [gefunden am 2014-08-04]
- ANONYMOUS, [Online] 15 März 2012, XP055224089 Gefunden im Internet: <URL:http://www.hpci-congress.com/index/hpc i_in/techse_in_abstracts.html> [gefunden am 2015-10-28]

## Beschreibung

Die vorliegende Erfindung betrifft eine kosmetische Zubereitung enthaltend Octyldodecylcitratpolyester, Dibutyladipat und Diethylhexyl Butamido Triazone.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Sonnenschutzmittel werden häufig in Zusammenhang mit Wassersportaktivitäten (Baden, Schwimmen, Surfen, Tauchen etc.) verwendet. Dabei tritt das Problem auf, dass sich das Sonnenschutzmittel, wenn die Haut noch feucht oder nass ist, nur schwer auf derselben applizieren lässt. In der Regel kommt es zum so genannten "Weißel-Effekt" bei dem Teile der Zubereitung beim Auftragen oder Verreiben auf der Haut ausfallen und nicht mehr auf die Haut aufziehen. Die Ausbildung eines gleichmäßigen Schutzfilms ist nicht mehr möglich. Das Ganze sieht dann nicht nur wenig ästhetisch aus. Derartig zerstörte kosmetische Systeme führen auch zu einem verminderten UV-Schutz, da die UV-Filter sich nicht mehr gleichmäßig auf der Haut verteilen und in diese einziehen können. Dieses Phänomen tritt vor allem bei Sonnenschutzölen und alkoholischen Lösungen auf, denn beim Auftrag auf die nasse Haut muss ja zuerst eine homogene Verbindung mit dem Wasser auf der Haut erfolgen. Anschließend muss sich diese homogene Mischung gleichmäßig auf der Haut verteilen lassen.

Es war daher die Aufgabe der vorliegenden Erfindung, den Mangel des Standes der Technik zu beseitigen und eine kosmetische Zubereitung, insbesondere ein Sonnenschutzmittel, zu entwickeln, dass sich einfach und stabil auf feuchte oder nasse Haut applizieren lässt und diese wirksam vor den Schäden des UV-Lichtes schützt ohne zu "weißeln".

Ferner haben Sonnenschutzmittel, die grundsätzlich für die Auftragung auf nasser Haut geeignet sind, den Nachteil, nicht besonders stabil zu sein sondern zu Trübungen (d.h. Ausfällungen von Rezepturbestandteilen) zu neigen.

Es war daher die Aufgabe der vorliegenden Erfindung, den Nachteil des Standes der Technik zu beseitigen und ein auf nasser Haut applizierbares Sonnenschutzmittel zu entwickeln, dass lager- und temperaturstabil ist und nicht zu Ausfällungen (= Trübungen der Zubereitung) neigt.

Überraschend gelöst werden die Aufgaben durch eine kosmetische Zubereitung enthaltend
a) Octyldodecylcitratpolyester (INCI: Octyldodecyl citrate crosspolymer mit der CAS-Nummer 1182066-69-8),
b) Dibutyladipat und
c) Diethylhexyl Butamido Triazone

Zwar kennt der Stand der Technik die WO 2012/009405, die US 2012/0014882 sowie das deutsche Gebrauchsmuster DE20 2013 001754.6, doch konnten diese Schriften nicht den Weg zur vorliegenden Erfindung weisen.

Im Rahmen der vorliegenden Offenbarung ist mit Zubereitung immer die erfindungsgemäße Zubereitung gemeint, wenn nichts anderes erwähnt ist.

Erfindungsgemäß vorteilhafte Ausführungsformen der vorliegenden Erfindung sind dadurch gekennzeichnet, dass die Zubereitung mindestens 2,5 Gewichts-%, bezogen auf das Gesamtgewicht der Zubereitung, Glycerin enthält.
Ein solcher Glycerin-Zusatz führt überraschenderweise dazu dass sich das Produkt beim Auftrag auf die Haut deutlich angenehmer anfühlt und weniger klebrig erscheint.

Es ist erfindungsgemäß vorteilhaft, wenn die Zubereitung Octyldodecylcitratpolyester in einer Konzentration von 7 bis Gewichts 12%, bezogen auf das Gesamtgewicht der Zubereitung, enthält.

Es ist erfindungsgemäß bevorzugt, wenn die Zubereitung Octyldodecylcitratpolyester in einer Konzentration von 8 bis 10 Gewichts-%, bezogen auf das Gesamtgewicht der Zubereitung, enthält.

Es ist vorteilhaft im Sinne der vorliegenden Erfindung, wenn die erfindungsgemäße Zubereitung Dibutyladipat in einer Konzentration von 3 bis 8 Gewichts-%, bezogen auf das Gesamtgewicht der Zubereitung, enthält.

Es ist bevorzugt im Sinne der vorliegenden Erfindung, wenn die erfindungsgemäße Zubereitung Dibutyladipat in einer Konzentration von 5 bis 7 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung Diethylhexyl Butamido Triazone in einer Konzentration von 0,3 bis 5 Gewichts-%, bezogen auf das Gesamtgewicht der Zubereitung, enthält.

Erfindungsgemäß bevorzugte Ausführungsformen der vorliegenden Erfindung sind dadurch gekennzeichnet, dass die Zubereitung Diethylhexyl Butamido Triazone in einer Konzentration von 0,5 bis 3 Gewichts-%, bezogen auf das Gesamtgewicht der Zubereitung, enthält.

Darüber hinaus ist es erfindungsgemäß von Vorteil, wenn die erfindungsgemäße Zubereitung einen oder mehrere UV-Filter enthält, gewählt aus der Gruppe der Verbindungen 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethyl-silyl)oxy]disiloxanyl]propyl]-phenol; 3-(4-Methylbenzyliden)campher; 3-Benzylidencampher; Ethylhexylsalicylat; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; 2,4-Bis-{[4-(2-ethyl-hexyloxy)-2-hydroxy]-phenyl}-6-(4-methoxyphenyl)-1,3,5-triazin (Bis- Ethylhexyloxyphenol methoxyphenyl Triazine); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate;Titandioxid; Zinkoxid.

Erfindungsgemäß besonders bevorzugte Ausführungsformen der vorliegenden Erfindung sind dadurch gekennzeichnet, dass die Zubereitung frei ist von 3-(4-Methylbenzyliden)-campher und 2-Hydroxy-4-methoxybenzophenon (Oxybenzon).

Hinsichtlich der Einsatzkonzentrationen der UV-Filter ist es erfindungsgemäß vorteilhaft, wenn die Zubereitung UV-Filter in der Gesamtmenge von 10 bis 40 Gewichts-%, bezogen auf das Gesamtgewicht der Zubereitung, enthält. Erfindungsgemäß bevorzugt ist es, wenn die Zubereitung UV-Filter in der Gesamtmenge von 12 bis 30 Gewichts-%, bezogen auf das Gesamtgewicht der Zubereitung, enthält.

Neben diesen lipophilen Komponenten kann die erfindungsgemäße Zubereitung auch noch weitere lipophile Bestandteile enthalten.

Es ist erfindungsgemäß von Vorteil, wenn die Zubereitung eine ethanolische Lösung darstellt.

In einem solchen Falle beträgt die erfindungsgemäß vorteilhafte Einsatzkonzentration des Ethanol von 20 bis 60 Gewichts-%, bezogen auf das Gesamtgewicht der Zubereitung.
Es ist erfindungsgemäß vorteilhaft, wenn die erfindungsgemäße kosmetische Zubereitung ein oder mehrere Parfümstoffe enthält. In einem solchen Falle ist es jedoch erfindungsgemäß von Bedeutung, dass die Zubereitung frei bleibt von Lyral. Erfindungsgemäß vorteilhafte Ausführungsformen der vorliegenden Erfindung sind dadurch gekennzeichnet, dass der Wassergehalt der Zubereitung weniger als 4,5 Gew.-% beträgt. Diese Angabe gilt natürlich nur für die Zubereitung vor dem Auftrag auf die Haut.

Es ist erfindungsgemäß von Vorteil, wenn die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Menthol, Panthenol, Taurin, Tocopherol, Tocopherolacetat, β-Alanin und/oder Licochalcon A, enthält.

Dabei ist es erfindungsgemäß bevorzugt, wenn die Zubereitung Panthenol in einer Konzentration von 0,5 bis 2 Gewichts-%, bezogen auf das Gesamtgewicht der Zubereitung, enthält.

Erfindungsgemäß bevorzugt handelt es sich bei der erfindungsgemäßen Zubereitung um ein kosmetisches Sonnenschutzmittel.

Die erfindungsgemäße Zubereitung kann erfindungsgemäß vorteilhaft in drei Ausführungsformen dargereicht werden:
So ist es in der ersten dieser Ausführungsformen erfindungsgemäß vorteilhaft, wenn die Zubereitung in Form eines Pumpsprays vorliegt. In einem solchen Fall können alle gängigen für die Kosmetik (insbesondere für sprühbare Sonnenschutzmittel) bekannten Pumpspray-Applikatoren eingesetzt werden.

Die zweite dieser vorteilhaften Ausführungsformen ist dadurch gekennzeichnet, dass die Zubereitung in Form eines Aerosolsprays vorliegt.

In einem solchen Falle ist es erfindungsgemäß vorteilhaft, wenn die Zubereitung als Treibgas Propan, n-Butan und/oder Isobutan enthält. Erfindungsgemäß bevorzugt sind dabei Mischungen dieser Gase.

Erfindungsgemäß bevorzugt wird die erfindungsgemäße Zubereitung in einer solchen Treibgasdose enthaltend die kosmetische Zubereitung, dargereicht.

Dabei ist es erfindungsgemäß bevorzugt, wenn die Dose eine Aluminiumdose ist, die auf der Innenseite mit einem Schutzlack beschichtet ist.

In einem solchen Falle ist es erfindungsgemäß vorteilhaft, wenn der Schutzlack ein Epoxyphenollack, ein Polyamid-Imid-Lack oder ein Pulverlack ist.
Es ist erfindungsgemäß vorteilhaft, wenn das Ventil der Treibgasdose im Sprühkopf ein Kugelventil enthält, das eine 360° Anwendung ermöglicht. Ein Beispiel hierfür sind Ariane Kugelventile der Firma Aptar.

Die Kegelbohrung kann erfindungsgemäß vorteilhaft 1x 0,32 mm oder 1x 0,44mm oder 1x 0,5mm betragen.
Darüber hinaus sind solche Treibgasdosen erfindungsgemäß vorteilhaft dadurch gekennzeichnet, dass als Sprühkopf z.B. Cindy von Lindal eingesetzt wird.

Die dritte dieser vorteilhaften Ausführungsformen ist dadurch gekennzeichnet, dass die Zubereitung in Form eines so genannten Bag-on-Valve- Sprays vorliegt. In einem solchen Fall können alle gängigen für die Kosmetik (insbesondere für sprühbare Sonnenschutzmittel) bekannten Bag-on-valve-Systemen eingesetzt werden.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **BSP.1** | **BSP. 2** | **BSP. 3** | **BSP. 4** | **BSP. 5** | **BSP. 6** | **BSP. 7** | **BSP. 8** |
|---|---|---|---|---|---|---|---|---|
| Panthenol | 0,5 | 1 | 1 | 0,5 | 1,5 | 0,5 | 2 | 2 |
| Dicaprylyl Ether | 5 | 2 | 2 | 5 | 2 | 3 | 5 | 5 |
| C12-15 Alkyl Benzoate | 2 | 11 | 5 | 8 | 11 | | 3 | 3 |
| Caprylic/Capric Triglycerides | 2 | | | | | | 4 | 4 |
| Paraffinum Liquidum | 3 | | | | | 4 | 4 | 4,5 |
| Dibutyl Adipate | 3 | 5 | 5 | 3 | 5 | 4 | 3 | 3 |
| Acrylates/Octylacrylamide Copolymer | 0,8 | 1,5 | 1,5 | 0,8 | 1,5 | 1 | 0,8 | 0,8 |
| Octyldodecyl Citrate Crosspolymer | 9 | 11 | 11 | 8 | 11 | 10 | 12 | 12 |
| Parfum | 0,5 | 0,5 | 0,5 | 0,5 | 0,5 | 0,3 | 1 | 1 |
| Glycerin | 2,5 | 3 | 3 | 2,5 | 3 | 5 | 4 | 4 |
| Alcohol Denat. + Aqua | 40 | 34,5 | 45 | 40 | 32,5 | 50 | 27 | 31 |
| Ethylhexyl Methoxycinnamate | 0,5 | 0,5 | 0,5 | 0,5 | 0,5 | 0,7 | 1 | 1 |
| Homosalate | 9 | 9 | 7 | 9 | 9 | 6 | 9,5 | 9,5 |
| Octocrylene | 9 | 9 | 7 | 9 | 9 | 6 | 9,5 | 9,5 |
| Ethylhexyl Salicylate | 4,5 | 4,5 | 4,5 | 4,5 | 4,5 | 4 | 4,5 | |
| Butyl Methoxydibenzoylmethane | 4,5 | | | 4,5 | 4,5 | 4 | 4,5 | 4,5 |
| Diethylhexyl Butamido Triazone | 2 | 1,5 | 1 | 2 | 1,5 | 1,5 | 2,2 | 2,2 |
| Bis-Ethylhexyloxyphenol Methoxyphenyl Triazine | | 2 | 2 | | 2 | | | |
| Polysilicone-15 | 0,7 | | | 0,7 | 1 | | 1,5 | 1,5 |
| Ethylhexyl Triazone | 1,5 | 1 | 1 | 1,5 | | | 1,5 | 1,5 |
| Diethylamino Hydroxybenzoyl Hexyl benzoate | | 3 | 3 | | | | | |

## Patentansprüche

1. Kosmetische Zubereitung enthaltend
a) Octyldodecylcitratpolyester (INCI: Octyldodecyl citrate crosspolymer mit der CAS-Nummer 1182066-69-8) und
b) Dibutyladipat,
c) Diethylhexyl Butamido Triazone.

2. Kosmetische Zubereitung nach Anspruch 1 **dadurch gekennzeichnet, dass** die Zubereitung mindestens 2,5 Gewichts-%, bezogen auf das Gesamtgewicht der Zubereitung, Glycerin enthält.

3. Kosmetische Zubereitung nach einem der vorhergehenden Ansprüche, **dadurch gekennzeichnet, dass** die Zubereitung Octyldodecylcitratpolyester in einer Konzentration von 7 bis 12 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Dibutyladipat in einer Konzentration von 3 bis 8 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung Diethylhexyl Butamido Triazone in einer Konzentration von 0,3 bis 5 Gewichts-%, bezogen auf das Gesamtgewicht der Zubereitung, enthält.

6. Kosmetische Zubereitung nach einem der vorhergehenden Ansprüche, **dadurch gekennzeichnet, dass** die Zubereitung einen oder mehrere UV-Filter enthält, gewählt aus der Gruppe der Verbindungen 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester ; 2,4-Bis-{[4-(2-ethyl-hexyloxy)-2-hydroxy]-phenyl}-6-(4-methoxyphenyl)-1,3,5-triazin (Bis- Ethylhexyloxyphenol methoxyphenyl Triazine); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate;Titandioxid; Zinkoxid.

7. Kosmetische Zubereitung nach einem der vorhergehenden Ansprüche, **dadurch gekennzeichnet, dass** die Zubereitung ein oder mehrere Parfümstoffe enthält.

8. Kosmetische Zubereitung nach einem der vorhergehenden Ansprüche, **dadurch gekennzeichnet, dass** der Wassergehalt der Zubereitung weniger als 4,5 Gew.-% beträgt.

9. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Menthol, Panthenol, Taurin, Tocopherol, Tocopherolacetat, β-Alanin und/oder Licochalcon A, enthält.

10. Kosmetische Zubereitung nach einem der vorhergehenden Ansprüche, **dadurch gekennzeichnet, dass** die Zubereitung Panthenol in einer Konzentration von 0,5 bis 2 Gewichts-%, bezogen auf das Gesamtgewicht der Zubereitung, enthält.

11. Kosmetische Zubereitung nach einem der vorhergehenden Ansprüche, **dadurch gekennzeichnet, dass** die Zubereitung in Form eines Pumpsprays vorliegt.

12. Kosmetische Zubereitung nach einem der Ansprüche 1 bis 11, **dadurch gekennzeichnet, dass** die Zubereitung in Form eines Aerosolsprays vorliegt.

13. Kosmetische Zubereitung nach Anspruch 12, **dadurch gekennzeichnet, dass** die Zubereitung als Treibgas Propan, n-Butan und/oder Isobutan enthält.

14. Kosmetische Zubereitung nach einem der vorhergehenden Ansprüche 1 bis 10, **dadurch gekennzeichnet, dass** die Zubereitung in Form eines Bag-on- Valve-System vorliegt.

## Claims

1. Cosmetic preparation comprising
a) octyldodecyl citrate polyester (INCI: Octyldodecyl citrate crosspolymer with the CAS number 1182066-69-8) and
b) dibutyl adipate,
c) Diethylhexyl Butamido Triazone.

2. Cosmetic preparation according to Claim 1, **characterized in that** the preparation comprises at least 2.5% by weight, based on the total weight of the preparation, of glycerol.

3. Cosmetic preparation according to one of the preceding claims, **characterized in that** the preparation comprises octyldodecyl citrate polyester in a concentration of from 7 to 12% by weight, based on the total weight of the preparation.

4. Cosmetic preparation according to one of the preceding claims, **characterized in that** the preparation comprises dibutyl adipate in a concentration of from 3 to 8% by weight, based on the total weight of the preparation.

5. Cosmetic preparation according to one of the preceding claims, **characterized in that** the preparation comprises Diethylhexyl Butamido Triazone in a concentration of from 0.3 to 5% by weight, based on the total weight of the preparation.

6. Cosmetic preparation according to one of the preceding claims, **characterized in that** the preparation comprises one or more UV filters selected from the group of the compounds 2,2'-methylenebis(6-(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; 4-(tert-butyl)-4'-methoxydibenzoylmethane; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethyl benzalmalonate; 3-(4-(2,2-bisethoxycarbonylvinyl)-phenoxy)propenyl)methoxysiloxane/dimethylsiloxane copolymer; hexyl 2-(4'-diethylamino-2'-hydoxy-benzoyl)benzoate; 2,4-bis{[4-(2-ethylhexyloxy)-2-hydroxy]phenyl}-6-(4-methoxyphenyl)-1,3,5-triazine(bis-ethylhexyloxyphenol methoxyphenyl triazine); 2,4-bis[5-1(dimethylpropyl)benzoxazol-2-yl-(4-phenyl)imino]-6-(2-ethylhexyl)imino-1,3,5-triazine with the (CAS No. 288254-16-0); tris(2-ethylhexyl) 4,4',4"-(1,3,5-triazine-2,4,6-triyl-triimino)trisbenzoate (also: 2,4,6-tris[anilino)p-carbo-2'-ethyl-1'-hexyloxy)]-1,3,5-triazine (INCI: Ethylhexyl Triazone); 2,4,6-tribiphenyl-4-yl-1,3,5-triazine; merocyanines; piperazine derivatives; titanium dioxide; zinc oxide.

7. Cosmetic preparation according to one of the preceding claims, **characterized in that** the preparation comprises one or more perfume substances.

8. Cosmetic preparation according to one of the preceding claims, **characterized in that** the water content of the preparation is less than 4.5% by weight.

9. Cosmetic preparation according to one of the preceding claims, **characterized in that** the preparation comprises one or more active ingredients selected from the group of the compounds glycyrrhetic acid, urea, arctiln, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatiinne, menthol, panthenol, taurine, tocopherol, tocopherol acetate, β-alanine and/or licochalcone A.

10. Cosmetic preparation according to one of the preceding claims, **characterized in that** the preparation comprises panthenol in a concentration of from 0.5 to 2% by weight, based on the total weight of the preparation.

11. Cosmetic preparation according to one of the preceding claims, **characterized in that** the preparation is present in the form of a pump spray.

12. Cosmetic preparation according to one of Claims 1 to 11, **characterized in that** the preparation is present in the form of an aerosol spray.

13. Cosmetic preparation according to Claim 12, **characterized in that** the preparation comprises propane, n-butane and/or isobutane as propellant gas.

14. Cosmetic preparation according to one of the preceding Claims 1 to 10, **characterized in that** the preparation is present in the form of a bag-on-valve system.

## Revendications

1. Préparation cosmétique contenant
a) du polyester de citrate d'octyldodécyle (INCI : Octyldodecyl citrate crosspolymer présentant le numéro CAS 1182066-69-8) et
b) de l'adipate de dibutyle,
c) de la diéthylhexylbutamidotriazone.

2. Préparation cosmétique selon la revendication 1, **caractérisée en ce que** la préparation contient au moins 2,5% en poids de glycérol, par rapport au poids total de la préparation.

3. Préparation cosmétique selon l'une quelconque des revendications précédentes, **caractérisée en ce que** la préparation contient du polyester de citrate d'octyldodécyle en une concentration de 7 à 12% en poids, par rapport au poids total de la préparation.

4. Préparation cosmétique selon l'une quelconque des revendications précédentes, **caractérisée en ce que** la préparation contient de l'adipate de dibutyle en une concentration de 3 à 8% en poids par rapport au poids total de la préparation.

5. Préparation cosmétique selon l'une quelconque des revendications précédentes, **caractérisée en ce que** la préparation contient de la diéthylhexylbutamidotriazone en une concentration de 0,3 à 5% en poids par rapport au poids total de la préparation.

6. Préparation cosmétique selon l'une quelconque des revendications précédentes, **caractérisée en ce que** la préparation contient un ou plusieurs filtres UV choisis dans le groupe des composés 2,2'-méthylènebis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; 4-(tert-butyl)-4'-méthoxydibenzoylméthane ; 2-cyano-3,3-diphénylacrylate de 2-éthylhexyl ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; salicylate d'homomenthyle ; 2-hydroxybenzoate de 2-éthylhexyle ; diméthicodiéthylbenzalmalonate ; copolymère de 3-(4-(2,2-bis-éthoxycarbonylvinyl)-phénoxy)propényl)-méthoxysiloxane/diméthylsiloxane ; ester hexylique de l'acide 2-(4'-diéthylamino-2'-hydroxybenzoyl)-benzoïque ; 2,4-bis-{[4-(2-éthylhexyloxy)-2-hydroxy]-phényl}-6-(4-méthoxyphényl)-1,3,5-triazine (Bis-Ethylhexyloxyphenol methoxyphenyl Triazine) ; 2,4-bis-[5-1(diméthylpropyl)benzoxazol-2-yl-(4-phényl)-imino]-6-(2-éthylhexyl)-imino-1,3,5-triazine présentant le n° CAS 288254-16-0 ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dérivés de pipérazine ; dioxyde de titane ; oxyde de zinc.

7. Préparation cosmétique selon l'une quelconque des revendications précédentes, **caractérisée en ce que** la préparation contient un ou plusieurs parfums.

8. Préparation cosmétique selon l'une quelconque des revendications précédentes, **caractérisée en ce que** la teneur en eau de la préparation est inférieure à 4,5% en poids.

9. Préparation cosmétique selon l'une quelconque des revendications précédentes, **caractérisée en ce que** la préparation contient une ou plusieurs substances actives choisies dans le groupe des composés acide glycyrrhétinique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, menthol, panthénol, taurine, tocophérol, acétate de tocophérol, ß-alanine et/ou licochalcone A.

10. Préparation cosmétique selon l'une quelconque des revendications précédentes, **caractérisée en ce que** la préparation contient du panthénol en une concentration de 0,5 à 2% en poids par rapport au poids total de la préparation.

11. Préparation cosmétique selon l'une quelconque des revendications précédentes, **caractérisée en ce que** la préparation se trouve sous forme d'un spray à pompe.

12. Préparation cosmétique selon l'une quelconque des revendications 1 à 11, **caractérisée en ce que** la préparation se trouve sous forme d'un spray d'aérosol.

13. Préparation cosmétique selon la revendication 12, **caractérisée en ce que** la préparation contient, comme gaz propulseur, du propane, du n-butane et/ou de l'isobutane.

14. Préparation cosmétique selon l'une quelconque des revendications précédentes 1 à 10, **caractérisée en ce que** la préparation se trouve sous forme d'un système valve à poche (Bag-on-Valve).
